# EUROPEAN PATENT APPLICATION

(11) **EP 4 610 349 A1**
(43) Date of publication of application: **03.09.2025**
(21) Application number: 23900691.9
(22) Date of filing: 06.12.2023
(51) Int. Cl.: C12N 5/10, C12N 5/0781, C12N 15/12, C12N 15/33, C12P 21/08

(54) **METHOD FOR PRODUCING IMMORTALIZED CELL**

(30) Priority: 06.12.2022 JP 2022195145
(71) Applicant: Kitayama Labes Co., Ltd., Ina City, Nagano 396-0025 (JP)
(72) Inventor: OHSUGI Itaru, Ina City, Nagano 3960025 (JP); TAKAI Naofumi, Ina City, Nagano 3960025 (JP)
(74) Representative: Vossius & Partner Patentanwälte Rechtsanwälte mbB
(86) International application number: PCT/JP2023/043649
(87) International publication number: WO 2024/122579

(57) **Abstract**

An object of the present invention is to provide a method for immortalizing an antibody-producing cell; and an immortalized transformed cell obtained by the method. The invention relates to a method for producing an immortalized cell, the method including introducing SV40 T antigen gene, genes of Bcl-2 family, and one or more genes selected from the group consisting of genes of Myc family and genes of cyclin D family into a non-immortalized antibody-producing cell to produce an immortalized cell; and a method for producing an immortalized antibody-producing cell, the method including collecting an antibody-producing cell from a mammal and subjecting the cell to primary culture; and introducing one or more genes selected from the group consisting of SV40 T antigen gene, genes of Bcl-2 family, genes of Myc family, and genes of cyclin D family into a primary antibody-producing cell thus obtained to produce an immortalized antibody-producing cell.

## Description

### TECHNICAL FIELD

The present invention relates to a method for immortalizing an antibody-producing cell, and an immortalized transformed cell obtained by the method.

Priority is claimed on Japanese Patent Application No. 2022-195145, filed on December 6, 2022, the content of which is incorporated herein by reference.

### BACKGROUND ART

Some cells have proliferative ability, while other cells do not proliferate. Even cells having proliferative ability are usually genetically limited in the number of times they can divide, and thus, have a lifespan. Therefore, for example, primary-cultured cells, which are cells collected from a tissue of an animal and cultured, can only divide a limited number of times even in a case where their cells proliferate under appropriate conditions, and then the cells lose the proliferative ability. As a result, for cells having useful traits, attempts have been made to achieve so-called immortalization, in which the number of times the cells can divide is not limited and the cells acquire the ability to divide without limitation. In general, cells acquire immortalization ability by transformation.

Monoclonal antibodies are widely used as a reagent for detecting a target substance using an immune reaction. In addition, in recent years, human monoclonal antibodies have been used as an active ingredient of antibody medicines, and the monoclonal antibodies have industrial value. In many cases, the monoclonal antibodies are produced by antibody-producing cells, and in order to stably supply the monoclonal antibodies, it is required to immortalize the antibody-producing cells such that the antibody-producing cells can be cultured in vitro at an industrial level without impairing the antibody-producing ability thereof.

A hybridoma method has already been established for production of monoclonal antibodies of rodents. The hybridoma method is one in which an animal is immunized, a single B cell that produces a desired monoclonal antibody is separated, primary-cultured, and fused with cancer cells of myeloma (myeloma) to establish the hybridoma. The hybridoma thus produced is immortalized while retaining the antibody-producing ability. However, since there is no appropriate hybridoma method other than that in rodents, it is necessary to perform transformation by a complicated genetic engineering method for immortalization of the antibody-producing cells.

As a method for immortalizing primary-cultured cells, for example, an immortalization method including introducing B-cell/CLL lymphoma 2 (Bcl-2) gene, Myc gene, and Ccnd1 gene, which are three kinds of cancer-related genes, into proB cell to perform transformation and immortalization is known (Non-Patent Document 1). In addition, a method for immortalizing human fetal fibroblasts by introducing c-Myc gene and simian virus 40 (SV40) large T antigen gene, which are two kinds of cancer-related genes, into the human fetal fibroblasts to transform the human fetal fibroblasts is known (Non-Patent Document 2).

### Citation List

### Non-Patent Documents

Non-Patent Document 1: Nakagawa, et al., Haematologica, 2011, vol. 96(9), p. 1318-1326.
Non-Patent Document 2: Kim, et al., Experimental and Molecular Medicine, 2001, vol. 33(4), p. 293-298.

### SUMMARY OF INVENTION

### Technical Problem

Unlike proB cells, an antibody-producing cell, which is a fully differentiated cell, cannot acquire immortalization ability even in a case where Bcl-2 gene, Myc gene, and Ccnd1 gene are introduced thereinto to perform transformation. In addition, in a case where c-Myc gene and SV40 large T antigen gene are introduced into an antibody-producing cell to perform transformation, the cell can be immortalized by acquiring the proliferative ability. However, the cell cannot retain the useful traits, and immortalized cells having the antibody-producing ability retained cannot be obtained.

An object of the present invention is to provide a method for immortalizing an antibody-producing cell, and an immortalized transformed cell obtained by the method. Solution to Problem

The present inventors have conducted intensive studies to accomplish the object, and as a result, they have found that an antibody-producing cell collected from a mammal immunized with an antigen can be immortalized while retaining the antibody-producing ability by introducing SV40 T antigen gene, genes of Myc family, genes of Bcl-2 family, and genes of cyclin D family into the antibody-producing cell to perform transformation, thereby completing the invention.

That is, the present invention provides the following drugs and the like.
[1] A method for producing an immortalized cell, the method including: introducing SV40 T antigen gene, genes of Bcl-2 family, and one or more genes selected from the group consisting of genes of Myc family and genes of cyclin D family into a non-immortalized antibody-producing cell to produce an immortalized cell.
[2] The method for producing an immortalized cell according to [1], in which the Myc family gene is c-Myc gene or L-Myc gene.
[3] The method for producing an immortalized cell according to [1] or [2], in which the genes of Bcl-2 family is Bcl-2 gene or Bcl-XL gene.
[4] The method for producing an immortalized cell according to any one of [1] to [3], in which the genes of cyclin D family is cyclin D1 gene or cyclin D3 gene.
[5] The method for producing an immortalized cell according to any one of [1] to [3], in which the SV40 T antigen gene, the genes of Bcl-2 family, the genes of Myc family, and the genes of cyclin D family are introduced into the non-immortalized antibody-producing cell.
[6] A method for producing an immortalized antibody-producing cell, the method including: collecting an antibody-producing cell from a mammal and subjecting the cell to primary culture; and introducing SV40 T antigen gene, genes of Bcl-2 family, and one or more genes selected from the group consisting of genes of Myc family and genes of cyclin D family into a primary antibody-producing cell thus obtained to produce an immortalized antibody-producing cell.
[7] The method for producing an immortalized antibody-producing cell according to [6], in which the mammal is an animal that has been immunized with an antigen, and an immortalized antibody-producing cell that produces an antibody recognizing the antigen is screened from the immortalized antibody-producing cell.
[8] A method for producing an antibody, the method including: producing an immortalized antibody-producing cell by the method for producing an immortalized antibody-producing cell according to [6] or [7]; and culturing the immortalized antibody-producing cell to recover an antibody thus produced.
[9] A transformed cell immortalized by introducing an exogenous SV40 T antigen gene, an exogenous genes of Bcl-2 family, and one or more genes selected from the group consisting of an exogenous genes of Myc family and an exogenous genes of cyclin D family into a non-immortalized antibody-producing cell.
[10] The transformed cell according to [9], in which the transformed cell has an antibody-producing ability.

### Advantageous Effects of Invention

According to the method for producing an immortalized cell of the invention, an antibody-producing cell obtained by primary subculture or the like can be relatively easily immortalized, and a transformed cell having acquired an immortalization ability while retaining an antibody-producing ability can be obtained. The transformed cell obtained in this manner is useful, for example, for production of a monoclonal antibody.

### BRIEF DESCRIPTION OF DRAWING

[FIG. 1] A cumulative population doubling of a transformed cell obtained by immortalizing a plasma cells producing an anti-rCRP rabbit monoclonal antibody collected from a rabbit in Example 1.
[FIG. 2] A view showing a temporal change in an amount of an anti-rCRP rabbit monoclonal antibody of a transformed cell obtained by immortalizing a plasma cells producing an anti-rCRP rabbit monoclonal antibody collected from a rabbit in Example 1.
[FIG. 3] An HPLC chromatogram of a culture supernatant of an immortalized transformed cell that produces an anti-rCRP rabbit monoclonal antibody in Example 1.
[FIG. 4] A view showing the results of SDS-PAGE of a purified anti-rCRP rabbit monoclonal antibody under a reduction condition (R in the figure) and a non-reduction condition (NR in the figure) in Example 1.
[FIG. 5] A view showing the results of Western blotting using a purified anti-rCRP rabbit monoclonal antibody in Example 1.
[FIG. 6] A view showing a cumulative population doubling of a transformed cell into which each expression cassette has been introduced in Example 2.
[FIG. 7] A view showing the results of the flow cytometry analysis of a cell group having transformed cells, into which each expression cassette has been introduced, stained with an anti-rabbit IgG antibody in Example 2.
[FIG. 8] A view showing a cumulative population doubling of a transformed cell into which each expression cassette has been introduced in Example 3.
[FIG. 9] A view showing a cumulative population doubling of a transformed cell into which each expression cassette has been introduced in Example 4.

### DESCRIPTION OF EMBODIMENTS

The method for producing an immortalized cell according to the present invention involves a method for producing an immortalized cell by introducing SV40 T antigen gene, genes of Bcl-2 family, and one or more genes selected from the group consisting of genes of Myc family and genes of cyclin D family into a non-immortalized antibody-producing cell. By introducing a combination of these cancer-related genes into the non-immortalized antibody-producing cell and expressing the combination, restriction on the number of cell divisions is lifted and cell division ability is imparted, whereby an immortalized cell can be obtained. Usually, differentiated plasma cells cannot divide in vitro, but by the method for producing an immortalized cell of the present invention, it is possible to artificially produce an immortalized cell having a doubling ability comparable to that of a cancer cell such as CHO cell.

In the method for producing an immortalized cell of the present invention, three genes of the SV40 T antigen gene, the genes of Bcl-2 family, and the genes of Myc family may be introduced into the non-immortalized antibody-producing cell, three genes of the SV40 T antigen gene, the genes of Bcl-2 family, and the genes of cyclin D family may be introduced into the non-immortalized antibody-producing cell, or four genes of the SV40 T antigen gene, the genes of Bcl-2 family, the genes of Myc family, and the genes of cyclin D family may be introduced into the non-immortalized antibody-producing cell. It is possible to impart an immortalization ability to the antibody-producing cells regardless of the combination. Since it is possible to produce a larger number of the transformed cells that acquired the immortalization ability while retaining the antibody-producing ability, in the method for producing an immortalized cell of the present invention, it is particularly preferable to introduce the four genes, that is, the SV40 T antigen gene, the genes of Bcl-2 family, the genes of Myc family, and the genes of cyclin D family.

The SV40 T antigen gene is a viral gene that is known to contribute to the immortalization of cells of many biological species by inactivating a tumor suppressor gene that induces cellular senescence. In the present invention and the specification of the present application, both the SV40 large T antigen and the SV40 small T antigen are collectively referred to as the "SV40 T antigen".

The "SV40 large T antigen" includes any of proteins having a function of inactivating a tumor suppressor gene that induces cellular senescence in the cell, similarly to a natural SV40 large T antigen. Furthermore, the term "natural" means a protein encoded in a genomic DNA of any biological species. Specifically, the SV40 large T antigen includes not only a protein consisting of the identical amino acid sequence as a natural SV40 large T antigen, but also a protein consisting of a mutant of the natural SV40 large T antigen, that is, an amino acid sequence in which one or more amino acids are deleted from, substituted with, or added to the identical amino acid sequence as the natural SV40 large T antigen, and which retains the tumor suppressor gene inactivation ability. In addition, the SV40 large T antigen encoded by the SV40 T antigen gene to be introduced into the non-immortalized cell in the invention may be a fusion protein in which various tags or other structural proteins are directly or indirectly linked to the natural SV40 large T antigen or a mutant thereof through an appropriate linker sequence. Furthermore, the amino acid sequence of the natural SV40 large T antigen is registered in the International Nucleotide Sequence Database (INSD) (INSD accession number: AAB59924).

The "SV40 small T antigen" includes any of proteins having a function of binding to protein phosphatase 2 (PP2A) to inactivate PP2A, similarly to the natural SV small T antigen. Specifically, the SV40 small T antigen includes not only a protein consisting of the identical amino acid sequence as natural SV40 small T antigen (INSD accession number: AAB59925), but also a protein which is a mutant of the natural SV40 small T antigen, that is, a protein consisting of an amino acid sequence in which one or more amino acids are deleted from, substituted with, or added to the identical amino acid sequence as the natural SV40 small T antigen, and which retains the PP2A inactivation ability. In addition, the SV40 small T antigen encoded by the SV40 T antigen gene to be introduced into the non-immortalized cell in the invention may be a fusion protein in which various tags or other structural proteins are directly or indirectly linked to the natural SV40 small T antigen or a mutant thereof through an appropriate linker sequence.

In the present invention and the specification of the present application, the "SV40 T antigen gene" is a gene encoding a protein having a function of inactivating a tumor suppressor gene that induces cellular senescence in a cell, similarly to the natural SV40 large T antigen, and means a nucleic acid including a nucleotide sequence encoding the SV40 T antigen. The "SV40 T antigen gene" used in the present invention may be a nucleic acid including both a nucleotide sequence encoding the SV40 large T antigen and a nucleotide sequence encoding the SV40 small T antigen, or may be a nucleic acid including only the nucleotide sequence encoding the SV40 large T antigen. The SV40 T antigen gene to be introduced into the non-immortalized cell in the present invention is not particularly limited as long as it is a gene capable of expressing the SV40 T antigen in a case where the gene is introduced into a mammalian cell, may be a gene including an intron and expressing both an SV40 large T antigen and an SV40 small T antigen, or may consist of only an exon. In addition, the nucleotide sequence encoding the SV40 large T antigen or the nucleotide sequence encoding the SV40 small T antigen in the SV40 T antigen gene may be subjected to various modifications such as a change from a degenerate codon to a nucleotide sequence with a high codon usage frequency in a finitely proliferative antibody-producing cell (which may hereinafter be simply referred to as a "finitely proliferative cell") into which the SV40 T antigen gene or the like is introduced for immortalization. The modification of the codon can be performed by a publicly-known gene sequence mutation techniques or artificial gene synthesis.

In the present invention and the specification of the present application, "Myc family" means a family of transcription factors including a basic helix-loop-helix (bHLH) and a leucine zipper (LZ) motif. The human Myc family has three transcription factors of c-Myc (INSD accession number: AAA36340), L-Myc (INSD accession number: BAG58834), and N-Myc (INSD accession number: AAP36048). In the invention and the specification of the application, the "Myc family" includes not only a natural Myc family, but also a modified form obtained by modifying the natural Myc family protein while retaining the transcription factor function in the same manner as in the human c-Myc. Specific examples of the Myc family include not only human c-Myc, human L-Myc, and human N-Myc, but also homologues thereof as the natural Myc family. Examples of the homologue of the human c-Myc include rabbit c-Myc (INSD accession number: AJC97784). Examples of the homologue of human L-Myc include rabbit L-Myc (INSD accession number: XP_002715238). In addition, the Myc family includes not only a protein consisting of the identical amino acid sequence as the natural Myc but also a mutant thereof, that is, a protein consisting of an amino acid sequence in which one or more amino acids have been deleted from, substituted with, or added to the identical amino acid sequence as the natural Myc, and which retains the transcriptional activity. Examples of the Myc family of the mutant include a T58N mutant of human c-Myc. In addition, the Myc family encoded by the Myc family gene to be introduced into the non-immortalized cell in the invention may be a modified form such as a fusion protein in which various tags or other structural proteins are linked directly or indirectly to a natural Myc family or a mutant thereof through an appropriate linker sequence. The genes of Myc family to be introduced into the non-immortalized cell in the invention is preferably a gene of c-Myc (human c-Myc and an orthologue thereof, and mutants thereof), L-Myc (human c-Myc and an orthologue thereof, and mutants thereof), or a modified form thereof, and more preferably the gene of human c-Myc, human L-Myc, or the mutant thereof, or the modified form thereof.

In the present invention and the specification of the present application, " genes of Myc family" means a nucleic acid including a nucleotide sequence encoding the Myc family. The genes of Myc family to be introduced into the non-immortalized cell in the invention is not particularly limited as long as it is a gene capable of expressing the Myc family in a case where the gene is introduced into a mammalian cell. The genes of Myc family may include an intron or may consist of only an exon. In addition, the nucleotide sequence encoding the Myc family proteins in the genes of Myc family may be subjected to various modifications such as a change from a degenerate codon to a codon with a high codon usage frequency in a finitely proliferative cell. The modification of the codon can be performed by a publicly-known gene sequence mutation techniques or artificial gene synthesis.

In the present invention and the specification of the present application, the "Bcl-2 family" means a family of mitochondrial outer membrane proteins which have one or more Bcl-2 homology (BH) domains and a membrane penetration region having high hydrophobicity on the C-terminal side, and have an anti-apoptotic action. Examples of the human Bcl-2 family include Bcl-2 (INSDA accession number: AAH27258), Bcl-XL (INSDA accession number: AAP35872), and MCL-1 (Myeloid cell leukemia sequence 1) (INSDA accession number: AAD13299) as family proteins having an anti-apoptotic action.

The genes of Bcl-2 family to be introduced into the non-immortalized cell in the present invention is not particularly limited as long as it is a protein which has one or more BH domains and a membrane penetration region, and has an anti-apoptotic action. For example, in the present invention and the specification of the present application, the "Bcl-2 family" includes not only a natural Bcl-2 family but also a modified form obtained by modifying the natural Bcl-2 family protein while retaining the anti-apoptotic action in the same manner as the human Bcl-2. Specific examples of the natural Bcl-2 family in the Bcl-2 families having an anti-apoptotic action include not only human Bcl-2, human Bcl-XL, and human MCL-1, but also homologues thereof. Examples of the homologue of the human Bcl-2 include rabbit Bcl-2 (INSD accession number: XP_008259661). Examples of the homologue of the human Bcl-XL include rabbit Bcl-XL (INSD accession number: XP_008254359). In addition, the Bcl-2 family protein includes not only a protein consisting of the identical amino acid sequence as the natural Bcl-2 family but also a protein consisting of a mutant thereof, that is, a protein consisting of an amino acid sequence in which one or more amino acids are deleted from, substituted with, or added to the identical amino acid sequence as the natural Bcl-2 family, and which retains the anti-apoptotic action. In addition, the Bcl-2 family encoded by the genes of Bcl-2 family to be introduced into the non-immortalized cell in the present invention may be a modified form such as a fusion protein in which various tags or other structural proteins are linked directly or indirectly to a natural Bcl-2 family or a mutant thereof through an appropriate linker sequence. The genes of Bcl-2 family to be introduced into the non-immortalized cell in the present invention is preferably a gene of Bcl-2 (human Bcl-2 and an orthologue thereof, and mutants thereof), Bcl-XL (human Bcl-XL and an orthologue thereof, and mutants thereof), or a modified form thereof, and more preferably the gene of the human Bcl-2, the human Bcl-XL, or mutants thereof, or modified forms thereof.

In the present invention and the specification of the present application, the term "genes of Bcl-2 family" means a nucleic acid including a nucleotide sequence encoding the Bcl-2 family. The genes of Bcl-2 family to be introduced into the non-immortalized cell in the present invention is not particularly limited as long as it is a gene capable of expressing the Bcl-2 family in a case where the gene is introduced into a mammalian cell. The genes of Bcl-2 family may include an intron or may consist of only an exon. In addition, the nucleotide sequence encoding the Bcl-2 family proteins in the genes of Bcl-2 family may be subjected to various modifications such as a change from a degenerate codon to a codon with a high codon usage frequency in a finitely proliferative cell. The modification of the codon can be performed by a publicly-known gene sequence mutation techniques or artificial gene synthesis.

In the present invention and the specification of the present application, "cyclin D family" means a family of proteins which have a cyclin box domain binding to a cyclin-dependent kinase (CDK) and a retinoblastoma tumor suppressor protein (RB) binding domain on the N-terminal side, and in which the synthesis is initiated in G1 phase of a cell cycle and a transition from G1 phase to S phase is controlled. Examples of the human cyclin D family include cyclin D1 (INSDA accession number: AAA58392), cyclin D2 (NSD accession number: AAA51926), and cyclin D3 (NSD accession number: AAA52137).

The cyclin D family gene to be introduced into the non-immortalized cell in the present invention is not particularly limited as long as it is a protein having the same S phase transition control action as that of the human cyclin D1. For example, in the present invention and the specification of the present application, the "cyclin D family" includes not only a natural cyclin D family but also a modified form obtained by modifying the natural cyclin D family protein while retaining the S phase transition control action. Specific examples of the natural cyclin D family include not only human cyclin D1, human cyclin D2, and human cyclin D3, but also homologues thereof. In addition, the cyclin D protein includes not only a protein consisting of the identical amino acid sequence as the natural cyclin D but also a mutant thereof, that is, a protein consisting of an amino acid sequence in which one or more amino acids have been deleted from, substituted with, or added to the identical amino acid sequence as the cyclin D, and which retains the S phase transition control action. In addition, the cyclin D family encoded by the genes of cyclin D family to be introduced into the non-immortalized cell in the present invention may be a modified form such as a fusion protein in which various tags or other structural proteins are directly or indirectly linked to a natural cyclin D family or a mutant thereof through an appropriate linker sequence. The genes of cyclin D family to be introduced into the non-immortalized cell in the present invention is preferably a gene of cyclin D1 (human cyclin D1 and an orthologue thereof, and mutants thereof), cyclin D3 (human cyclin D3 and an orthologue thereof, and mutants thereof), or a modified form thereof, and more preferably the gene of the human cyclin D1, the human cyclin D3, or the mutant thereof, or the modified form thereof.

In the method for producing an immortalized cell of the present invention, the genes of Myc family, the genes of Bcl-2 family, and the genes of cyclin D family among the genes to be introduced into the non-immortalized cell may be genes derived from the same biological species as the cells to be introduced, or may be genes derived from different biological species. In addition, these three kinds of genes may be genes derived from the same biological species or may be genes derived from different biological species. For example, the SV40 T antigen gene, the human c-Myc gene, the human Bcl-2 gene, and the human cyclin D1 gene may be introduced into lymph node cells collected from a rabbit; the SV40 T antigen gene, the mouse c-Myc gene, the human Bcl-2 gene, and the human cyclin D1 gene may be introduced into spleen cells collected from a mouse; and the SV40 T antigen gene, the human c-Myc gene, the human Bcl-2 gene, and the human cyclin D1 gene may be introduced into epithelial cells collected from a human.

In the method for producing an immortalized cell of the present invention, the cell to be subjected to transformation for immortalization is not particularly limited as long as it is a non-immortalized, finitely proliferative cell. The biological species, the tissue, and the like from which the cell is derived are not particularly limited. The finitely proliferative cell provided for the present invention is preferably a cell collected from an animal or a primary-cultured cell obtained by culturing the cell, and more preferably a cell collected from a mammal or a primary-cultured cell obtained by culturing the cell. The mammal is not particularly limited, but is more preferably human, an experimental animal such as mouse, rat, and monkey, domestic animal or pet such as rabbit, dog, cat, cow, horse, and sheep, and particularly preferably human. The collection and the primary culture of cells from the animal can be performed by ordinary methods.

In the method for producing an immortalized cell of the present invention, a non-immortalized plasma cells are preferably used as the cell to be subjected to transformation for immortalization. Here, the plasma cells are mononuclear cells similar to lymphocytes distributed in the spleen, the lymph node, the bone marrow, the connective tissue, and the like, and acquires an antibody-producing ability by the stimulation of an antigen. The plasma cells to be subjected to transformation of the present invention may be any of a plasma cells before acquiring an antibody-producing ability, a plasma cells retaining an antibody-producing ability after acquiring the antibody-producing ability, or a plasma cells having lost an antibody-producing ability after acquiring the antibody-producing ability. The plasma cells can be sorted by those skilled in the art by appropriately selecting means such as flow cytometry. For the confirmation of the selected plasma cells, the plasma cells may be confirmed by hematoxylin-eosin staining, the shape of the nucleus, and the image of the plasma cells by an optical microscope in the cytoplasm.

In the method for producing an immortalized cell of the present invention, an immortalized transformed cell can be produced by introducing an SV40 T antigen gene, genes of Myc family, genes of Bcl-2 family, and genes of cyclin D family into a non-immortalized cell by a genetic engineering method. All of these four kinds of genes may be introduced simultaneously or sequentially (in no particular order) into the non-immortalized cell.

In the method for producing an immortalized cell of the present invention, SV40 T antigen gene and the like may be introduced into a chromosome of the cells to be immortalized, or may be introduced as an extrachromosomal gene. A transformed cell having excellent maintenance stability of subculture is obtained by introducing SV40 T antigen gene or the like into a chromosome.

The genetic engineering method used for introducing the SV40 T antigen gene and the like into the antibody-producing cell to be immortalized may be any publicly-known method of introducing a foreign gene into a finitely proliferative cell, and a method obtained by appropriately modifying a publicly-known method can also be used. Examples of the method include a method of introducing a vector, into which an expression cassette of a foreign gene has been incorporated, into a cell. The SV40 T antigen gene and the like may be expressed in a polycistronic type or may be expressed in a monocistronic type.

The expression cassette is a combination of DNAs required for expressing a target protein, and includes a structural gene encoding the target protein and a promoter that functions in a finitely proliferative cell. Only one kind of structural gene or two or more kinds of structural genes may be included in a single expression cassette.

In a case of monocistronic expression, specific examples of the expression cassette used in transformation for immortalization include an expression cassette for SV40 T antigen, which includes SV40 T antigen gene and a promoter, an expression cassette for Myc family, which includes genes of Myc family and a promoter, an expression cassette for Bcl-2 family, which includes genes of Bcl-2 family and a promoter, and an expression cassette for cyclin D family, which includes genes of cyclin D family and a promoter.

Two or more kinds of the SV40 T antigen gene, the genes of Myc family, the genes of Bcl-2 family, and the genes of cyclin D family may be incorporated into a single expression cassette for polycistronic expression. For example, a transformed cell that has been immortalized can also be obtained by introducing a single expression cassette into a finitely proliferative cell, the single expression cassette being obtained by sequentially linking an SV40 T antigen gene, genes of Myc family , genes of Bcl-2 family, and genes of cyclin D family to each other downstream of a single promoter through a linker sequence that includes a nucleotide sequence encoding a self-cleaving peptide. As the self-cleaving peptide, a publicly-known self-cleaving peptide such as P2A peptide, E2A peptide, and T2A peptide can be appropriately selected and used. The linker sequence that links each gene may include an internal ribosome entry site (IRES) sequence instead of the self-cleaving peptide. The IRES sequence may be an IRES sequence derived from a virus genome or IRES sequence derived from an animal cell genome, and can be appropriately selected from known IRES sequences.

The promoter in the expression cassette may be any promoter that functions in a finitely proliferative cell, may be a promoter that is originally possessed by a finitely proliferative cell, or may be a promoter that is not originally possessed by a finitely proliferative cell. In a case where the finitely proliferative cell is an antibody-producing plasma cells, examples of the promoter that is originally possessed by a finitely proliferative cell include a promoter of Blimp1 gene (Blimp1 promoter) that is specifically expressed in a plasma cells. Examples of the promoter that is not originally possessed by the finitely proliferative cell include a promoter derived from an animal cell virus and an artificial promoter obtained by modifying the promoter. As the promoter used in the invention, Piggyback promoter, CAG promoter, CBh promoter, SV40 promoter, SRα promoter, hCMV promoter, or the like is preferable from the viewpoint of widespread and extensive track record of use. All the promoters of the SV40 T antigen gene and the like may be the same as or different from each other.

The expression cassette may further include any one or more of a terminator that functions in a finitely proliferative cell, an enhancer that functions in a finitely proliferative cell, 5'-untranslated region, and 3'-untranslated region. As the terminator, the terminator that is originally possessed by a finitely proliferative cell or a terminator that is not originally possessed by a finitely proliferative cell can be used. Examples of the enhancer include an Eu enhancer.

As a vector into which an expression cassette including SV40 T antigen gene and the like is incorporated, a non-viral vector such as a plasmid vector can be used. The plasmid vector can be produced by incorporating the expression cassette into a vector having a circular DNA structure. A vector having a linear DNA structure into which an expression cassette is incorporated can also be used instead of the plasmid vector.

In a case where a transformant in which the expression cassette is retained as an extrachromosomal gene in a finitely proliferative cell is created, the vector is preferably a plasmid including a sequence for replication in the finitely proliferative cell, that is, an autonomously replicating sequence (ARS). On the other hand, in a case where a transformant in which the expression cassette has been incorporated into a chromosome of a finitely proliferative cell is created, the expression cassette is preferably a vector having a linear DNA structure having no ARS. The expression cassette may be a plasmid vector having no ARS, the plasmid vector including a restriction enzyme recognition sequence for cleaving the linear DNA during the introduction of the vector a finitely proliferative cell.

In a case where the vector is introduced into a chromosome of a finitely proliferative cell, the vector has homologous recombination sites consisting of nucleotide sequence that enables homologous recombination to occur with respect to a target site for homologous recombination in the chromosome of the finitely proliferative cell, upstream and downstream of the expression cassette in the vector. The homologous recombination site can be appropriately set based on the nucleotide sequence information of the genomic DNA of the finitely proliferative cell. The target site for homologous recombination in the chromosome of the finitely proliferative cell may be only one site in the chromosome of the finitely proliferative cell, or a plurality of sites in the chromosome may be used as the target site. In a case where a plurality of sites in the chromosome are used as the target site, it is preferable that a transposon be used as the target site. For example, a transformed cell (antibody-producing cell) that has been immortalized is obtained by introducing a vector into which an expression cassette for polycistronic expression, including at least one gene of the SV40 T antigen gene, the genes of Bcl-2 family, the genes of Myc family, and the genes of cyclin D family, between a 5' transposon-specific repeat sequence and a 3' transposon-specific repeat sequence, has been incorporated into a finitely proliferative cell, and the expression cassette is incorporated into a plurality of transposons present in the chromosome of the finitely proliferative cell.

In a case where the vector into which the expression cassette including the SV40 T antigen gene and the like is incorporated is a non-viral vector, the introduction into the finitely proliferative cell can be performed by a method appropriately selected from publicly-known transfection methods such as an electroporation method, a microinjection method, a lipofection method, and a calcium phosphate method.

The vector into which the expression cassette including the SV40 T antigen gene and the like is incorporated may be a viral vector. As the viral vector, various publicly-known viral vectors used for gene introduction into primary-cultured cells, such as a lentiviral vector, an adeno-associated viral vector, and a retroviral vector, can be used. Synthesis of each viral vector and subsequent production of a virus into which an expression cassette including the SV40 T antigen gene and the like has been incorporated can be performed by an ordinary method, using a commercially available virus expression kit.

The culture of the finitely proliferative cell before and during the introduction of the vector into which the expression cassette including the SV40 T antigen gene and the like is incorporated, and the culture of the transformed cell obtained after the introduction of the vector can be performed under the same culture conditions using the same culture medium as those used for the culture of the finitely proliferative cell subjected to immortalization.

By the method for producing an immortalized cell of the present invention, an immortalized transformed cell having an antibody-producing ability can be produced. The biological species of the antibody-producing cell is preferably a mammal, and more preferably animal species used for production of a monoclonal antibody that is used as a reagent or the like in the related art, such as human, mouse, rat, rabbit, donkey, horse, sheep, and goat.

For example, a transformed cell producing an immortalized antibody can be produced by collecting an antibody-producing cell from a mammal and subjecting the cell to primary culture; and introducing at least one gene of SV40 T antigen gene, genes of Bcl-2 family, genes of Myc family, and genes of cyclin D family into a primary antibody-producing cell thus obtained. In addition, in the body of a patient suffering from an infectious disease, an antibody against a causative microorganism of the infectious disease is produced. Therefore, by introducing at least one gene of the SV40 T antigen gene, the genes of Bcl-2 family, the genes of Myc family, and the genes of cyclin D family into an antibody-producing plasma cells collected from the patient suffering from an infectious disease, it is possible to obtain a transformed cell that has acquired immortalization ability while retaining the antibody-producing ability against the causative microorganism of the infectious disease.

For example, a mammal is immunized with an antigen, an antibody-producing cell is formed in the body of the animal, and then the cell is collected from a tissue, for example, a lymph node, which includes the antibody-producing cell. At least one gene of SV40 T antigen gene, genes of Bcl-2 family, genes of Myc family , and genes of cyclin D family is introduced into a population of the collected lymph node cells to obtain immortalized transformed cells. From the obtained population of the immortalized transformed cells, a transformed cell that produces an antibody recognizing an antigen used for immunization (immortalized antibody-producing cell) is screened. In this manner, an immortalized antibody-producing cell that produces an antibody recognizing a target antigen is obtained. By culturing the immortalized antibody-producing cell thus obtained, and thus collecting the produced antibody, an antibody against a target antigen can be stably produced. The antibody produced by the immortalized antibody-producing cell can be used as it is as a research reagent in the same manner as with other antibodies, and can also be used as raw materials for pharmaceutical compositions, hygiene products (masks and the like), contamination removal-related products, and the like.

### Examples

Next, the present invention will be described in more detail with reference to Examples, but the present invention is not limited to Examples.

### [Example 1]

Anti-rCRP antibody-producing B cells (antibody-producing cells) were collected from rabbits immunized with C-reactive protein (rCRP), and these were immortalized to obtain transformed cells.

### (1) Creation of Immunogen and Screening Antigen

rCRP (manufactured by Oriental Yeast Co., Ltd.) was used as an immunogen. In addition, the same antigen was used as an antigen for screening in ELISA which will be used later.

### (2) Animal and Antigen Immunization Method

As the immunization animal, female rabbits (Japanese white rabbit, 2.5 to 3.0 kg) were used. The immunization was performed at an interval of 2 weeks with an emulsion created by mixing the antigen and adjuvant in equal amounts. The primary immunization was performed using a Freund's complete adjuvant as the adjuvant such that the amount of the immunogen per rabbit was 1 mg. The second and subsequent immunizations were performed using a Freund's incomplete adjuvant as the adjuvant such that the amount of the immunogen per rabbit was 0.5 mg.

### [2] Gene Introduction and Screening

### (1) Production of Expression Vector

An expression cassette A (SEQ ID NO: 1: CAG promoter at the 1st to 1,679th, human cyclin D1 gene at the 1,734th to 2,618th, P2A peptide-encoding nucleotide sequence at the 2,619th to 2,684th, rabbit Bcl-2 gene at the 2,685th to 3,362nd, P2A peptide-encoding nucleotide sequence at the 3,363rd to 3,428th, rabbit c-Myc gene (including stop codon) at the 3,429th to 4,745th, and bovine growth hormone-polyA signal at the 4,789th to 5,013rd), in which the human cyclin D1 gene, the rabbit Bcl-2 gene, and the rabbit c-Myc gene had been sequentially linked through a linker sequence including a P2A peptide that is a self-cleaving peptide, was incorporated downstream of the CAG promoter of a mammalian gene expression vector (manufactured by Vector Builder, Inc.). The linking was performed using a seamless cloning method. Further, as an expression cassette for the SV40 T antigen gene, an expression cassette B (SEQ ID NO: 2: an SV40 promoter at the 1st to 330th, large T antigen exon at the 344th to 2,816th, small T antigen exon at the 344th to 868th, a large T antigen intron at the 590th to 935th, and SV40 polyA signal at the 2,839th to 2,973rd) obtained by excising a region including the SV40 promoter, the SV40 T antigen gene, and the SV40 polyA signal from the SV40 virus DNA was prepared and incorporated upstream of the CAG promoter of the expression vector.

### (2) Collection of Antibody-Producing Cell

The blood was collected from the immunized animal over time and an antibody titer of the anti-rCRP antibody in the serum was measured by ELISA. After a sufficient increase in antibody titer was confirmed, the popliteal lymph nodes were excised and a suspension of popliteal lymph node cells was prepared according to an ordinary method.

### (3) Gene Introduction

A plasmid vector into which the expression cassette A had been incorporated was introduced into the popliteal lymph node cells by an electroporation method. The cell suspension of the popliteal lymph node cells after gene introduction, and the subsequent culture were suspended in RPMI1640 medium (manufactured by Sigma-Aldrich Co., LLC) including 1% by mass methyl cellulose (manufactured by Nacalai Tesque, Inc.), 30% by volume fetal bovine serum (FBS), and 1% by mass penicillin/streptomycin, and cultured in 100 mm dishes.

### (4) Cell Proliferation

The 100 mm dishes were cultured by being left to stand in an incubator in wet state at 37°C and 5% by volume CO₂ for 14 days. The cells that proliferated and formed colonies in the culture medium are transformed cells into which genes have been introduced, and are monoclonal cells. Each colony was collected from the 100 mm dishes with a stereomicroscope using a pipette during culture, transferred to RPMI 1640 medium (manufactured by Sigma-Aldrich Co. LLC) including 10% FBS and 1% P/S, and cultured in microtiter plates until confluent.

### (5) ELISA

The anti-rCRP antibody in the culture supernatant obtained by culturing the transformed cells was detected by an ELISA method.

First, rCRP (0.5 µg/mL) was immobilized on an ELISA plate, and then the ELISA plate was blocked with 1% by mass BSA. Thereafter, the culture supernatant of the 100 mm dishes, in which the medium had been replaced the previous day, was dispensed into the ELISA plate and incubated for a predetermined time to perform the reaction. As a result, the anti-rCRP rabbit monoclonal antibody in the culture supernatant was bound to rCRP immobilized in each well of the ELISA plate.

Next, horse radish peroxidase-labeled anti-rabbit IgG antibody was dispensed into each well of the ELISA plate and incubated for a predetermined time to dispense the peroxidase, which is an enzyme substrate, and the fluorescence intensity was measured with a plate reader to detect the transformed cells producing the anti-rCRP rabbit monoclonal antibody.

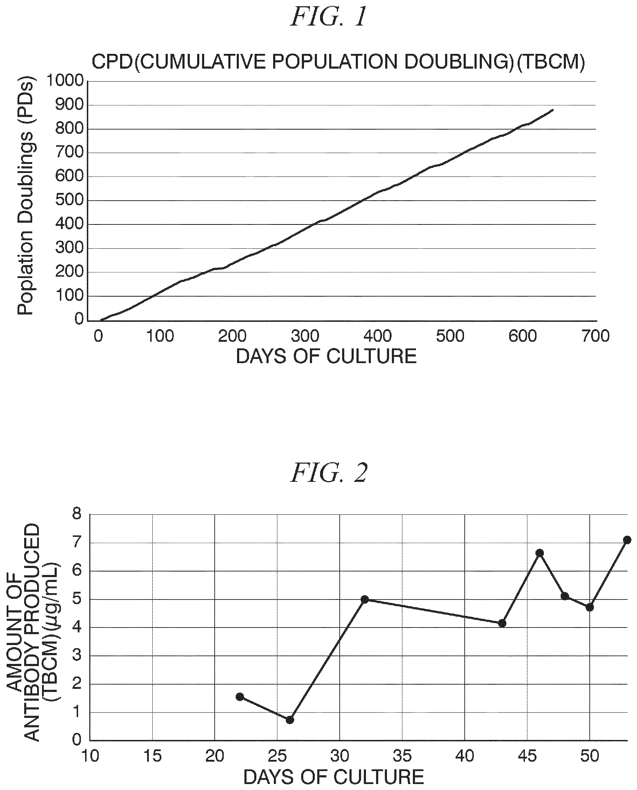

The obtained transformed cells were subcultured three times a week in 6-well plates for a week in order to confirm the proliferative ability and the antibody-producing ability. The number of cells of the transformed cells and the amount of the anti-rCRP rabbit monoclonal antibody were measured over time. The number of the transformed cells was counted under a microscope. The amount of the anti-rCRP rabbit monoclonal antibody was measured by the same ELISA as described above. A cumulative population doubling of the number of the transformed cells is shown in FIG. 1, and the measurement results of the amount (µg/mL) of the anti-rCRP rabbit monoclonal antibody are shown in FIG. 2.

As a result, as shown in FIG. 1, the obtained transformed cells proliferated for nearly 650 days. In addition, the doubling time was approximately 16 hours, and a decline in the proliferation rate was not observed. That is, in the present Example, it was possible to artificially produce an immortalized cell of a suspension cell having the same degree of doubling ability as cancer cells such as CHO cells. In addition, as shown in FIG. 2, it was found that the obtained transformed cells produced the anti-rCRP rabbit monoclonal antibody for nearly 60 days, and transformed cells that were immortalized while retaining the antibody-producing ability were obtained just by introducing the SV40 T antigen gene, the genes of Myc family, the genes of Bcl-2 family, and the genes of cyclin D family into the primary-cultured antibody-producing cells.

### [3] Purification and Electrophoresis of Monoclonal Antibody

### (1) Purification

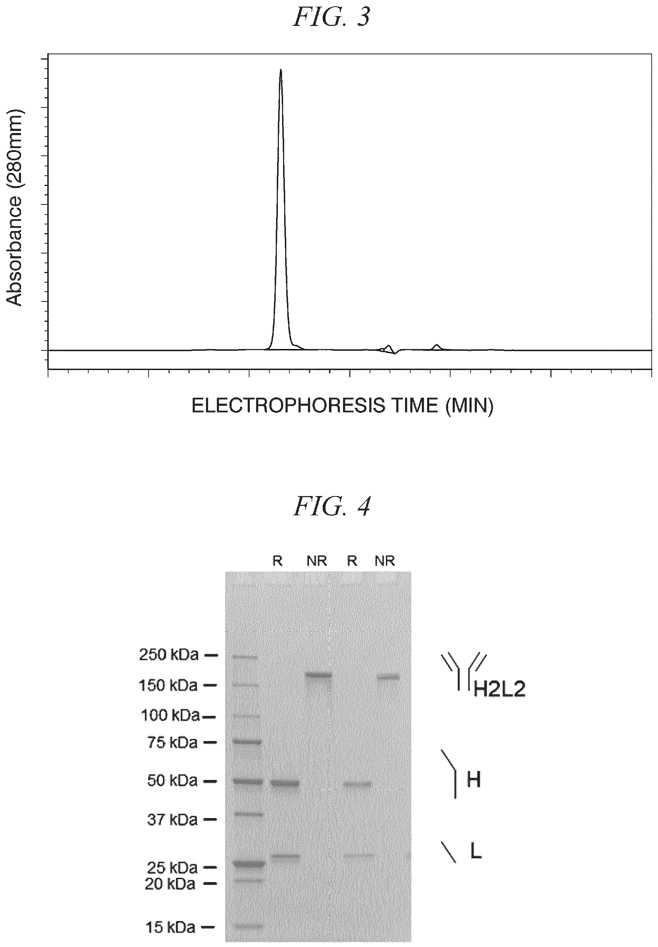

Expansion culture was performed using transformed cells having an antibody-producing ability. The culture conditions were the same manner as described above. Thereafter, the anti-rCRP antibody was purified from the culture supernatant obtained according to an ordinary method using a Protein A column. The purified antibody was subjected to HPLC at flow rate of 0.7 mL/min using a column for size exclusion chromatography (SEC) (TSKgel G3000SWXL, manufactured by Tosoh Corporation). A chromatogram of HPLC is shown in FIG. 3. In the chromatogram, it was confirmed that there was only one peak and there was no problem in the purity.

### (2) SDS-PAGE

SDS-PAGE was performed according to a standard method using the purified antibody. "Perfect Nt Gel" (manufactured by DRC Co., Ltd.) was used as gel, 1 µg of the purified antibody was used, and "Optibopt Blue" (manufactured by Abcam Limited) was used as staining solution. The antibody that had been subjected to reduction treatment (reducing conditions) and the antibody that had not been subjected to the reduction treatment (non-reducing conditions) were each subjected to SDS-PAGE. The results of SDS-PAGE are shown in FIG. 4. In FIG. 4, "R" is a lane through which the antibody that had been subjected to the reduction treatment was applied, and "NR" is a lane through which the antibody that had not been subjected to the reduction treatment was applied. The band at approximately 160 kDa is a band of a complete antibody (an antibody consisting of two heavy chains (H chains) and two light chains (L chains)), the band at approximately 50 kDa is a band of one H chain, and the band at approximately 25 kDa is a band of one L chain. As a result, it was confirmed that the band was obtained at a target molecular weight under both the reducing conditions and the non-reducing conditions.

### (3) Western Blotting

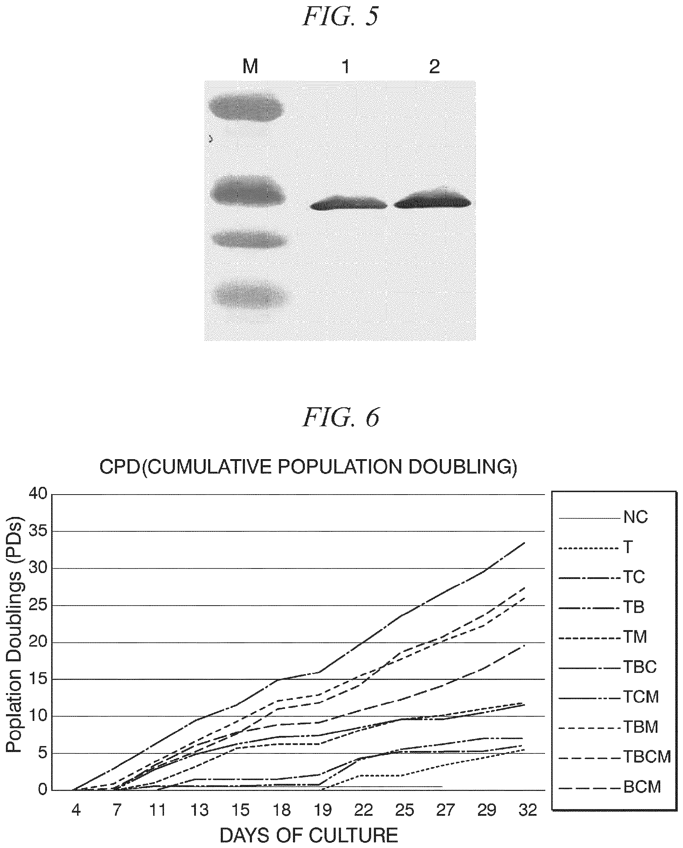

rCRP used as the immunogen was subjected to SDS-PAGE using the purified antibody, and transferred to a PVDF membrane, and then the obtained PVDF membrane was subjected to Western blotting according to an ordinary method. The PVDF membrane was "iBlot Gel Transfer Stacks PVDF, MINI" (manufactured by Invitrogen Corporation), the blocking liquid was "Blocking One" (manufactured by Nakalai Tesque, Inc.), the secondary antibody was "Anti-IgG, rabbit, Goat-Poly, HRP" (manufactured by GeneTex, Inc.), and the coloring liquid was "KPL TMB Membrane Peroxidase Substrate (1-c)" (manufactured by SeraCare Life Sciences, Inc.). The results of the Western blotting are shown in FIG. 5. In FIG. 5, lane 1 is a lane through which "Anti-C Reactive Protein Antibody [Y284] (ab32412)" (manufactured by Abcam Limited) was applied as a positive control,lane 2 is a lane through which a purified antibody sample was applied, and lane M is a lane through which a molecular weight marker was applied. As shown in FIG. 5, the purified antibody bound to the band of rCRP in the PVDF membrane and developed color.

### (4) Sequence of Antibody Gene

In order to confirm that the antibody produced by the immortalized transformed cells is a monoclonal antibody, the sequence of the antibody gene was confirmed. First, RNA was extracted from the cell and cDNA was synthesized by a reverse transcription reaction. Extraction of RNA from the immortalized transformed cell was performed using "NucleoSpin (registered trademark) RNA", and the reverse transcription reaction was performed using "PrimeScript IV 1st strand cDNA Synthesis Mix" (manufactured by Takara Bio Inc.). Next, cDNA was used as a template, and PCR was performed using a primer set (Table 1) specific for each of the H chain and the L chain of the rabbit antibody gene, and a polymerase ("PrimeSTAR Max DNA Polymerase", manufactured by Takara Bio Inc.), thereby amplifying the antibody gene.

**[Table 1]**

| | | Nucleotide sequence | SEQ ID NO. |
|---|---|---|---|
| For H chain of rabbit IgG | Forward primer | CTCACCATGGAGACTGGGCTG | 3 |
| | Reverse primer | TCATTTACCCGGAGAGCGGGA | 4 |
| For L chain of rabbit IgG | Forward primer | AGGCAGGACCCAGCATGGACA | 5 |
| | Reverse primer | ACAGTCACCCCTATTGAAGC | 6 |

Each of the obtained PCR fragments was purified using "NucleoSpin" (manufactured by TaKaRa Bio Inc.), and then the nucleotide sequence was confirmed by a direct sequencing method using the primer described in Table 1. As a result, it was found that the cDNA of the H chain of the anti-rCRP antibody produced by the immortalized transformed cells consisted of the nucleotide sequence set forth in SEQ ID NO: 7 and the cDNA of the L chain of the antibody consisted of the nucleotide sequence set forth in SEQ ID NO: 9. From the results of these nucleotide sequences, it was confirmed at the genetic level that the anti-rCRP antibody produced by the immortalized transformed cell is a monoclonal antibody from the amino acid sequence of the H chain (SEQ ID NO: 8: a signal sequence at the 1st to 19th , a frame 1 at the 20th to 43rd , CDR1 at the 44th to 51st, a frame 2 at the 52nd to 68th, CDR2 at the 69th to 75th, a frame 3 at the 76th to 111th, CDR3 at the 112th to 130th a frame 4 at the 131st to 141st, and a γ chain constant region at the 142nd to 464th), and the amino acid sequence of the L chain (SEQ ID NO: 10: a signal sequences at the 1st to 22nd, a frame 1 at the 23rd to 48th bases, CDR1 at the 49th to 55th, a frame 2 at the 56th to 72nd , CDR2 at the 73rd to 75th, a frame 3 at the 76th to 111th, CDR3 at the 112th to 123rd, a frame 4 at the 124th to 133rd, and a κ chain constant region at the 134th to 237th).

### [Example 2]

The SV40 T antigen gene, the genes of Bcl-2 family, the genes of Myc family, and the genes of cyclin D family were introduced in combination into primary-cultured cells of rabbit spleen cells, and the effects on the immortalization ability and the antibody-producing ability were examined.

### [1] Gene Introduction and Screening

### (1) Production of Expression Vector

The expression cassette of each gene was incorporated into one plasmid vector in a combination shown in Table 2 to prepare an expression vector. In Table 2, "O" in the column of each expression cassette indicates that the expression cassette was incorporated into the expression vector. The expression vector NC is a vector (control vector) into which none of the expression cassettes were incorporated.

**[Table 2]**

| | Expression cassette | | | |
|---|---|---|---|---|
| Name of expression vector | Cyclin D 1 | Bc1-2 | c-Myc | SV40 T Antigen |
| NC | | | | |
| T | | | | ○ |
| TB | | ○ | | ○ |
| TC | ○ | | | O |
| TM | | | ○ | ○ |
| TBC | ○ | ○ | | ○ |
| TBM | | ○ | ○ | ○ |
| TCM | ○ | | ○ | ○ |
| TBCM | ○ | ○ | ○ | ○ |
| BCM | ○ | ○ | ○ | |

The expression vector T was produced by using the expression cassette B used in Example 1 as the expression cassette of the SV40 T antigen gene, and incorporating the expression cassette B into the control vector.

The expression vector TB was produced by incorporating an expression cassette of rabbit Bcl-2 gene (SEQ ID NO: 11: CAG promoter at the 1st to 1,679th, rabbit Bcl-2 gene (including stop codon) at the 1,734th to 2,414th , and bovine growth hormone-polyA signal at the 2,458th to 2,682nd ), and the expression cassette of the SV40 T antigen gene (expression cassette B) into the control vector.

The expression vector TC was produced by incorporating an expression cassette of human cyclin D1 gene (SEQ ID NO: 12: CAG promoter at the 1st to 1,679th human cyclin D1 gene (including stop codon) at the 1,734th to 2,621st , and bovine growth hormone-polyA signal at the 2,665th to 2,889th ), and the expression cassette of the SV40 T antigen gene (expression cassette B) into the control vector.

The expression vector TM was produced by incorporating an expression cassette of rabbit c-Myc gene (SEQ ID NO: 13: CAG promoter at the 1st to 1,679th , rabbit c-Myc gene (including stop codon) at the 1,734th to 3,050th , and bovine growth hormone-polyA signal at the 3,094th to 3,318th ), and the expression cassette of the SV40 T antigen gene (expression cassette B) into the control vector.

The expression vector TBC was produced by incorporating an expression cassette (SEQ ID NO: 14: CAG promoter at the 1st to 1,679th, human cyclin D1 gene at the 1,734th to 2,618th, P2A peptide-encoding nucleotide sequence at the 2,619th to 2,684th, a rabbit Bcl-2 gene (including stop codon) at the 2,685th to 3,365th, and bovine growth hormone-polyA signal at the 3,409th to 3,633rd of the human cyclin D1 gene and the rabbit Bcl-2 gene, and the expression cassette of the SV40 T antigen gene (expression cassette B) into the control vector.

The expression vector TBM was produced by incorporating an expression cassette (SEQ ID NO: 15: CAG promoter at the 1st to 1,679th bases, rabbit Bcl-2 gene at the 1,734th to 2,411st P2A peptide-encoding nucleotide sequence at the 2,412nd to 2,477th , rabbit c-Myc gene (including stop codon) at the 2,478th to 3,794th, and bovine growth hormone-polyA signal at the 3,838th to 4,062nd) of the rabbit Bcl-2 gene and the rabbit c-Myc gene, and the expression cassette of the SV40 T antigen gene (expression cassette B) into the control vector.

The expression vector TCM was produced by incorporating an expression cassette (SEQ ID NO: 16: CAG promoter at the 1st to 1,679th, human cyclin D1 gene (including stop codon) at the 1,734th to 2,618th, P2A peptide-encoding nucleotide sequence at the 2,619th to 2,684th, rabbit c-Myc gene (including stop codon) at the 2,685th to 4,001st , and bovine growth hormone-polyA signal at the 4,045th to 4,269th of the human cyclin D1 gene and the rabbit c-Myc gene, and the expression cassette of the SV40 T antigen gene (expression cassette B) into the control vector.

The expression vector TBCM was produced by incorporating the expression cassette of the human cyclin D1 gene, the rabbit Bcl-2 gene, and the rabbit c-Myc gene (expression cassette A used in Example 1), and the expression cassette of the SV40 T antigen gene (expression cassette B) into the control vector.

The expression vector BCM was produced by incorporating the expression cassette of the human cyclin D1 gene, the rabbit Bcl-2 gene, and the rabbit c-Myc gene (expression cassette A) into the control vector.

### (2) Gene Introduction

The spleen was excised from the rabbit and a suspension of spleen cells was prepared according to an ordinary method. Each expression vector was introduced into the spleen cells by an electroporation method. The cell suspension of the spleen cells after gene introduction and the subsequent culture were performed in the same manner as the popliteal lymph node cells after the electroporation of Example 1, and the transformed cells into which the genes had been introduced proliferated and then formed colonies.

### (3) Evaluation of Cell Proliferative Ability

Each colony was collected from the 100 mm dishes with a stereomicroscope using a pipette during culture, transferred to RPMI 1640 medium (manufactured by Sigma-Aldrich Co. LLC) including 10% FBS and 1% P/S*,* and the amount of the cells in the medium was measured by counting the number under a microscope. A cumulative population doubling based on the measurement results of the cumulative number of the cells measured over time after gene introduction by electroporation is shown in FIG. 6.

As shown in FIG. 6, in the transformed cells into which the expression vector NC (control vector) had been introduced, the proliferation was not confirmed until the 27th day of culture after gene introduction. In contrast, in the transformed cells into which the expression cassette of at least any of the four genes had been introduced, the proliferation of the cells was confirmed at 32nd day of culture, and it was confirmed that the plasma cells (antibody-producing cells) that did not proliferate in vitro originally acquired the immortalization ability. In addition, from FIG. 6, it was confirmed that the cell proliferation rate varieds depending on the kind and the combination of the introduced genes. The proliferation of the expression vector T and the expression vector TB was not confirmed until the 19th day of culture, the proliferation of the expression vector TC was not confirmed until the 12th day of culture, and the proliferation rate was considerably slow. The transformed cells into which the expression vectors (TBC, TBCM, TBM, BCM) including the expression cassette of the Bcl-2 gene had been introduced had a significantly faster cell proliferation rate than the transformed cells into which the expression vector (TM, TCM) not including the expression cassette of the Bcl-2 gene had been introduced, and in particular, the transformed cells into which the expression vector TBC, the expression vector TBCM, or the expression vector TBM had been introduced had a high proliferative ability.

### (4) Confirmation of Antibody-Producing Cells

The cumulative cell proliferation curve shown in FIG. 6 was obtained by measuring the proliferation of all cells obtained by transforming the collected spleen cells, and also includes cells other than the plasma cells (antibody-producing cells). Thus, the proportion of the antibody-producing cells in the cell group after culturing for 19, 25, or 32 days after gene introduction was examined by a flow cytometry method. Specifically, the transformed cells into which each expression cassette had been introduced were subjected to immobilization and a cell membrane permeation treatment using a membrane permeation treatment reagent "PerFix nc" (manufactured by BECKMAN COULTER, INC.), and stained with a fluorescently labeled goat anti-rabbit IgG antibody ("Goat Anti Rabbit IgG H & L (Alexa Fluor (registered trademark) 647, manufactured by Abcam Limited). Since the antibody-producing cells contain endogenous IgG in the cytoplasm, the cells were fluorescently stained with a fluorescently labeled goat anti-rabbit IgG antibody. The stained cells were analyzed by flow cytometry, and a proportion (%) of the fluorescently stained cells (antibody-producing cells) to the total number of cells was determined. The flow cytometry was performed using a flow cytometer "BD Accuri (registered trademark) C6 Plus" (manufactured by Becton, Dickinson and Company).

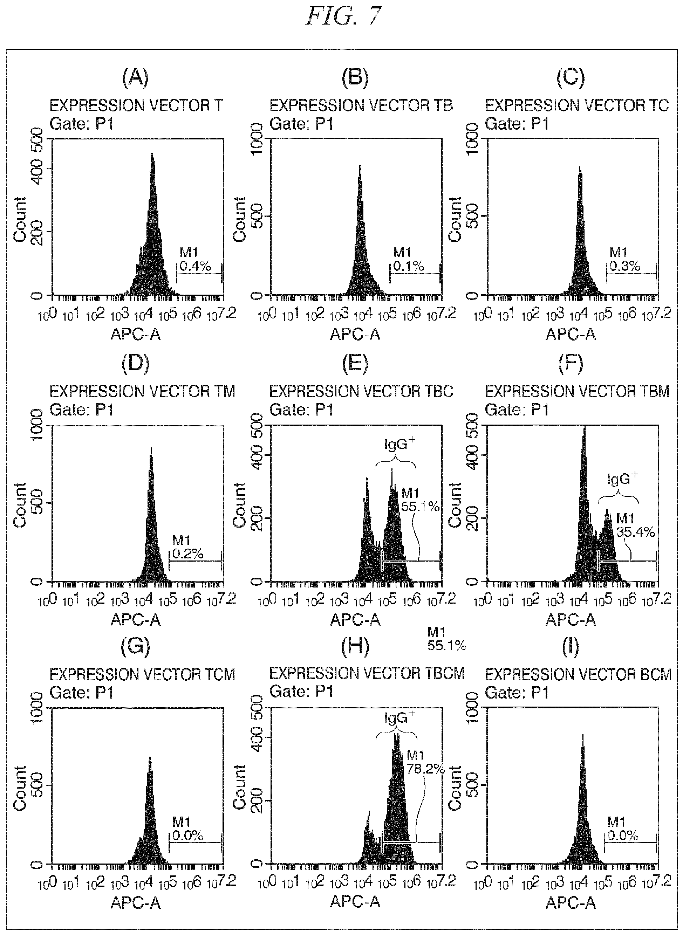

The results of the flow cytometry of the cells after culturing for 32 days after gene introduction are shown in FIG. 7. (A) to (I) of FIG. 7 are each the results of the flow cytometry of a transformed cell group into which the expression vector T, the expression vector TB, the expression vector TC, the expression vector TM, the expression vector TBC, the expression vector TBM, the expression vector TCM, the expression vector TBCM, and the expression vector BCM were each introduced. In the figure, "M1" indicates a cell group fluorescently stained with a fluorescently labeled goat anti-rabbit IgG antibody, that is, an antibody-producing cell group.

The results of the calculation of a proportion (IgG ratio) (%) of the antibody-producing cells from the results of the flow cytometry are shown in Table 3. As a result, the proliferation of the antibody-producing cells was confirmed only in the transformed cells into which the expression vector TBC, the expression vector TBCM, or the expression vector TBM had been introduced. In this manner, it was found that it is possible to immortalize the antibody-producing cell while retaining the antibody-producing ability by introducing the SV40 T antigen gene, the genes of Bcl-2 family, and one or more genes selected from the group consisting of the genes of Myc family and the genes of cyclin D family into the antibody-producing cell. In particular, it was found that in the cells into which the expression vector TBCM had been introduced, that is, the cells into which all of the four genes of the SV40 T antigen gene, the genes of Bcl-2 family, the genes of Myc family, and the genes of cyclin D family had been introduced, the proportion of the antibody-producing cells was large, and that the introduction of these four genes enableds effective production of immortalized antibody-producing cells.

**[Table 3]**

| Expression vector (gene introduced) | IgG ratio (%) | | |
|---|---|---|---|
| | 19th Day of culture | 25th Day of culture | 32nd Day of culture |
| T | N/A | N/A | 0.4 |
| TB | N/A | 0.6 | 0.1 |
| TC | N/A | 2.3 | 0.3 |
| TM | N/A | 0.8 | 0.2 |
| TBC | 57.9 | 59.1 | 55.1 |
| TBM | 7.3 | 27.3 | 35.4 |
| TCM | 0.7 | 0.3 | 0.0 |
| TBCM | 38.2 | 77.6 | 78.2 |
| BCM | 0.4 | 1.2 | 0.0 |

### [Example 3]

In Example 2, the effect of substitution of one gene selected from the group consisting of the genes of Bcl-2 family, the genes of Myc family, and the genes of cyclin D family with another gene in the gene family on the immortalization ability and the antibody-producing ability was examined.

### [1] Gene Introduction and Screening

### (1) Production of Expression Vector

The expression cassette of each gene was incorporated into one plasmid vector in a combination shown in Table 4 to prepare an expression vector. In Table 4, "O" in the column of each expression cassette indicates that the expression cassette was incorporated into the expression vector. The expression vector NC is a vector (control vector) into which none of the expression cassettes were incorporated.

**[Table 4]**

| | Expression cassette | | | | | | |
|---|---|---|---|---|---|---|---|
| Name of expression vector | Cyclin D1 | Cyclin D3 | Bcl-2 | Bc1-XL | c-Myc | 1-Myc | SV40 T Antigen |
| NC | | | | | | | |
| TBCM | ○ | | ○ | | ○ | | ○ |
| TB'CM | ○ | | | ○ | O | | ○ |
| TBC'M | | ○ | ○ | | ○ | | ○ |
| TBCM' | ○ | | ○ | | | ○ | ○ |

The expression vector TBCM was produced in the same manner as in the method for the expression vector TBCM described in Example 2.

The expression vector TB'CM was produced by incorporating an expression cassette (SEQ ID NO: 17: CAG promoter at the 1st to 1,679th, human cyclin D1 gene at the 1,734th to 2,618th P2A peptide-encoding nucleotide sequence at the 2,619th to 2,684th, human Bcl-XL gene at the 2,685th to 3,383rd, P2A peptide-encoding nucleotide sequence at the 3,384th to 3,449th, a rabbit c-Myc gene (including stop codon) at the 3,450th to 4,766th and bovine growth hormone-polyA signal at the 4,810 to 5,034th) of the human cyclin D1 gene, the human Bcl-XL gene, and the rabbit c-Myc gene, and the expression cassette of the SV40 T antigen gene (expression cassette B) into the control vector.

The expression vector TBC'M was produced by incorporating an expression cassette (SEQ ID NO: 18: CAG promoter at the 1st to 1,679th, human cyclin D3 gene at the 1,734th to 2,609th, P2A peptide-encoding nucleotide sequence at the 2,610th to 2,675th, a rabbit Bcl-2 gene at the 2,676th to 3,353rd, P2A peptide-encoding nucleotide sequence at the 3,354th to 3,419th, rabbit c-Myc gene (including stop codon) at the 3,420th to 4,736th, and bovine growth hormone-polyA signal at the 4,780th to 5,004th ) of the human cyclin D3 gene, the rabbit Bcl-2 gene, and the rabbit c-Myc gene, and the expression cassette of the SV40 T antigen gene (expression cassette B) into the control vector.

The expression vector TBCM' was produced by incorporating an expression cassette (SEQ ID NO: 19: CAG promoter at the 1st to 1,679th human cyclin D1 gene at the 1,734th to 2,618th, P2A peptide-encoding nucleotide sequence at the 2,619th to 2,684th, rabbit Bcl-2 gene at the 2,685th to 3,362nd a P2A peptide-encoding nucleotide sequence at the 3,363rd to 3,428th, rabbit L-Myc gene (including stop codon) at the 3,429th to 4,613rd, and bovine growth hormone-polyA signal at the 4,657th to 4,881st) of the human cyclin D1 gene, the rabbit Bcl-2 gene, and the rabbit L-Myc gene, and the expression cassette of the SV40 T antigen gene (expression cassette B) into the control vector.

### (2) Gene Introduction

Each gene was introduced into rabbit spleen cells in the same manner as in the method described in Example 2 to obtain a transformed cells.

### (3) Evaluation of Cell Proliferative Ability

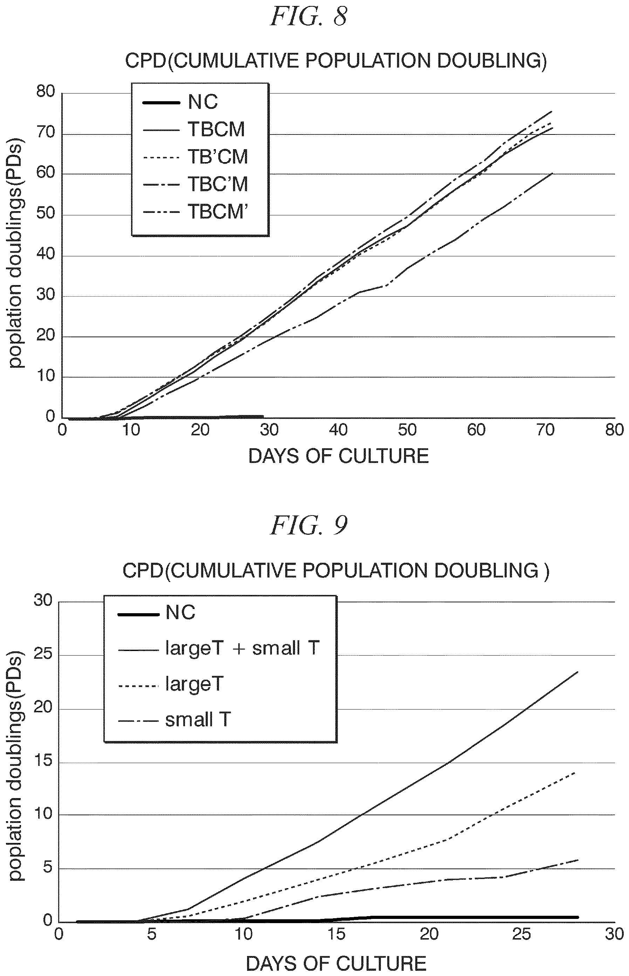

The cell proliferative ability was evaluated by measuring each cell amount of the transformed cells over time after gene introduction in the same manner as in the method described in Example 2. A cumulative population doubling based on the measurement results is shown in FIG. 8.

As shown in FIG. 8, in the transformed cells into which the expression vector NC had been introduced, the proliferation was not confirmed until the 29th day of culture after gene introduction. In contrast, in the transformed cells into which the expression vector (TB'CM, TBC'M, TBCM') had been introduced, the proliferation of the cells was confirmed as in the transformed cells into which the expression vector (TBCM) had been introduced, and it was confirmed that the cells acquired the immortalization ability.

In addition, from FIG. 8, it was confirmed that the cell proliferation rate varieds depending on the kind and the combination of the introduced genes, as in Example 2. The transformed cells into which the expression vector (TB'CM, TBC'M) had been introduced exhibited the same cell proliferation rate as the transformed cells into which the expression vector (TBCM) had been introduced. The transformed cells into which the expression vector (TBCM') had been introduced had a slightly slower cell proliferation rate than the transformed cells into which the expression vector (TBCM, TB'CM, TBC'M) had been introduced, but the cell proliferation rate was not a problem for commercial use.

### (4) Confirmation of Antibody-Producing Cells

The cumulative cell proliferation curve shown in FIG. 8 was obtained by measuring the proliferation of all cells obtained by transforming the collected spleen cells, and also includes cells other than the plasma cells (antibody-producing cells). Thus, the proportion of the antibody-producing cells in the cell group after culturing for 14, 21, or 28 days after gene introduction was examined by a flow cytometry method. Specifically, the method was performed in the same manner as in the method described in Example 2.

The results of the calculation of a proportion (IgG ratio) (%) of the antibody-producing cells from the results of the flow cytometry are shown in Table 5. As a result, the proliferation of the antibody-producing cells was confirmed in all the combinations. From these results, it was found that it is possible to perform immortalization while retaining the antibody-producing ability in any of a case where the Bcl-2 family gene to be used has a substitution of the Bcl-2 gene with the Bcl-XL gene, a case where the Myc family gene to be used has a substitution of the c-Myc gene with the L-Myc gene, and a case where the cyclin D family gene to be used has a substitution of the cyclin D1 gene with the cyclin D3 gene. In other words, it was clarified that the genes of Bcl-2 family, the genes of Myc family, and the genes of cyclin D family used in the present invention can immortalize a plasma cells while retaining the antibody-producing ability, even in a case where any gene in each gene family is used.

**[Table 5]**

| Expression vector (gene introduced) | IgG ratio (%) | | |
|---|---|---|---|
| | 14th Day of culture | 21st Day of culture | 28th Day of culture |
| TBCM | 70.3 | 46.3 | 52.1 |
| TB'CM | 58.7 | 60.5 | 57.5 |
| TBC'M | 64.2 | 47.1 | 49.3 |
| TBCM' | 51.5 | 21.3 | 32.1 |

### [Example 4]

In any of a case where the SV40 T antigen gene of the expression vector TBCM in Example 2 was changed to only the SV40 large T antigen gene, a case where the SV40 T antigen gene was changed to only the SV40 small T antigen gene, and a case where the SV40 T antigen gene was changed to both the SV40 large T antigen gene and the SV40 small T antigen gene, the effect on the immortalization ability was examined.

### [1] Gene Introduction and Screening

### (1) Production of Expression Vector

The expression cassette of each gene was incorporated into one plasmid vector in a combination shown in Table 6 to prepare an expression vector. In Table 6, "O" in the column of each expression cassette indicates that the expression cassette was incorporated into the expression vector. The expression vector NC is a vector (control vector) into which none of the expression cassettes were incorporated.

**[Table 6]**

| | Expression cassette | | | | |
|---|---|---|---|---|---|
| Name of expression vector | Cyclin D1 | Bc1-2 | c-Myc | SV40 Large T antigen | SV40 Small T antigen |
| NC | | | | | |
| large T + small T | ○ | ○ | ○ | ○ | ○ |
| large T | ○ | ○ | ○ | ○ | |
| small T | ○ | ○ | O | | ○ |

The expression vector (large T + small T) was produced in the same manner as in the method for the expression vector TBCM described in Example 2.

The expression vector (large T) was produced by incorporating the expression cassette of the human cyclin D1 gene, the rabbit Bcl-2 gene, and the rabbit c-Myc gene (expression cassette A used in Example 1), and an expression cassette of an SV40 large T antigen gene (SEQ ID NO: 20: SV40 promoter at the 1st to 330th, large T antigen-encoding gene (including stop codon) at the 344th to 2,470th and SV40 polyA signal at the 2,493rd to 2,627th) into the control vector.

The expression vector (small T) was produced by incorporating the expression cassette of the human cyclin D1 gene, the rabbit Bcl-2 gene, and the rabbit c-Myc gene (expression cassette A used in Example 1), and an expression cassette of an SV40 T antigen gene (SEQ ID NO: 21: SV40 promoter at the 1st to 330th, small T antigen-encoding gene (including a stop codon) at the 344th to 868th, and SV40 polyA signal at the 891st to 1,025th) into the control vector.

### (2) Gene Introduction

Each gene was introduced into rabbit spleen cells in the same manner as in the method described in Example 2 to obtain a transformed cell.

### (3) Evaluation of Cell Proliferative Ability

The cell proliferative ability was evaluated by measuring each cell amount of the transformed cells over time after gene introduction in the same manner as in the method described in Example 2. A cumulative population doubling based on the measurement results is shown in FIG. 9.

As shown in FIG. 9, in the transformed cells into which the expression vector NC had been introduced, the proliferation was not confirmed until the 28th day of culture after gene introduction. In contrast, in any of the transformed cells into which the expression vector (large T + small T, large T, and small T) had been introduced, the proliferation of the cells was confirmed, and it was confirmed that the cells acquired the immortalization ability.

From FIG. 9, it was confirmed that the cell proliferation rate varieds depending on the kind and the combination of the introduced genes. Specifically, it was confirmed that the cell proliferation rate was the highest in the transformed cells into which the expression vector (large T + small T) had been introduced, and the cell proliferation rate was different in the order of the expression vector (large T + small T) > the expression vector (large T) > the expression vector (small T). In the transformed cells into which the expression vector (small T) had been introduced, the proliferation was finally confirmed from around the 10th day of culture. In a case where only the SV40 small T antigen gene had been introduced, the proliferation was promoted, but was very slow. From these results, it is clear that the SV40 large T antigen gene is preferably included as the SV40 T antigen gene to be introduced into the plasma cells in the invention, the SV40 large T antigen gene may be included alone, but both the SV40 large T antigen gene and the SV40 small T antigen gene are particularly preferably included.

## Claims

1. A method for producing an immortalized cell, the method comprising:
introducing SV40 T antigen gene, genes of Bcl-2 family, and one or more genes selected from the group consisting of genes of Myc family and genes of cyclin D family into a non-immortalized antibody-producing cell to produce an immortalized cell.

2. The method for producing an immortalized cell according to Claim 1, wherein the genes of Myc family is c-Myc gene or L-Myc gene.

3. The method for producing an immortalized cell according to Claim 1, wherein the genes of Bcl-2 family is Bcl-2 gene or Bcl-XL gene.

4. The method for producing an immortalized cell according to Claim 1, wherein the genes of cyclin D family is cyclin D1 gene or cyclin D3 gene.

5. The method for producing an immortalized cell according to Claim 1,
wherein the SV40 T antigen gene, the genes of Bcl-2 family, the genes of Myc family, and the genes of cyclin D family are introduced into the non-immortalized antibody-producing cell.

6. A method for producing an immortalized antibody-producing cell, the method comprising:
collecting an antibody-producing cell from a mammal and subjecting the cell to primary culture; and
introducing SV40 T antigen gene, genes of Bcl-2 family, and one or more genes selected from the group consisting of genes of Myc family and genes of cyclin D family into a primary antibody-producing cell thus obtained to produce an immortalized antibody-producing cell.

7. The method for producing an immortalized antibody-producing cell according to Claim 6,
wherein the mammal is an animal that has been immunized with an antigen, and
an immortalized antibody-producing cell that produces an antibody recognizing the antigen is screened from the immortalized antibody-producing cell.

8. A method for producing an antibody, the method comprising:
producing an immortalized antibody-producing cell by the method for producing an immortalized antibody-producing cell according to Claim 6 or 7; and
culturing the immortalized antibody-producing cell to recover an antibody thus produced.

9. A transformed cell immortalized by introducing an exogenous SV40 T antigen gene, an exogenous genes of Bcl-2 family, and one or more genes selected from the group consisting of an exogenous genes of Myc family and an exogenous genes of cyclin D family into a non-immortalized antibody-producing cell.

10. The transformed cell according to Claim 9,
wherein the transformed cell has an antibody-producing ability.
